(19) Europäisches Patentamt
European Patent Office
Office européen des brevets

(11) Publication number: **0 565 794 A1**

(12) **EUROPEAN PATENT APPLICATION**

(21) Application number: **92307692.1**

(51) Int. Cl.$^5$: **A61K 39/00**

(22) Date of filing: **21.08.92**

(30) Priority: **14.04.92 GB 9208218**
**17.06.92 GB 9212816**

(43) Date of publication of application:
**20.10.93 Bulletin 93/42**

(84) Designated Contracting States:
**AT BE CH DE DK ES FR GB GR IE IT LI LU MC NL PT SE**

(71) Applicant: **BRITISH BIO-TECHNOLOGY LIMITED**
**Watlington Road**
**Cowley Oxford OX4 5LY(GB)**

(72) Inventor: **Layton, Guy Timothy, c/o British Bio-techn. Ltd.**
**Watlington Road**
**Cowley, Oxford OX4 5LY(GB)**
Inventor: **Burns, Nigel Robert, c/o British Bio-techn. Ltd.**
**Watlington Road**
**Cowley, Oxford OX4 5LY(GB)**
Inventor: **Adams, Sally Elizabeth, c/o British Bio-techn. Ltd**
**Watlington Road**
**Cowley, Oxford OX4 5LY(GB)**
Inventor: **Kingsman, Alan John, c/o British Bio-techn. Ltd**
**Watlington Road**
**Cowley, Oxford OX4 5LY(GB)**
Inventor: **Kingsman, Susan Mary, c/o British Bio-techn. Ltd**
**Watlington Road**
**Cowley, Oxford OX4 5LY(GB)**
Inventor: **Harris, Stephen John, c/o British Bio-techn. Ltd**
**Watlington Road**
**Cowley, Oxford OX4 5LY(GB)**
Inventor: **Gearing, Andrew J.H., c/o British Bio-techn. Ltd**
**Watlington Road**
**Cowley, Oxford OX4 5LY(GB)**

(74) Representative: **Sheard, Andrew Gregory et al**
**Kilburn & Strode**
**30, John Street**
**London WC1N 2DD (GB)**

# (54) Induction of CTL responses.

(57) Pharmaceutical formulations for use as immunotherapeutic or prophylactic vaccines comprise a proteinaceous particulate antigen made up of a plurality of self-assembling protein molecules. To enhance the cytotoxic T-lymphocyte (CTL) response, the formulations are adjuvant free. As adjuvant is desirable for enhancing the antibody response, a subject may be administered with two formulations, one with and one without adjuvant.

EP 0 565 794 A1

This invention relates to the induction of specific cytotoxic T cell responses in a host by immunisation and to pharmaceutical formulations for use in such induction.

Following viral infection, a mammalian host mounts both antibody and cytotoxic T cell (CTL) responses, and the subsequent protection against viruses from the same group is long-lived. However, although local or systemic antibodies acting alone can neutralise infectivity and block the spread of free cytolytic viruses released from host cells, they are incapable of controlling infection once the virus has penetrated and infected host cells. Although antibody dependent cell-mediated cytotoxicity (ADCC) or antibody-dependent complement-mediated lysis may be mechanisms whereby infected cells expressing viral antigens can be destroyed, the CTL response is thought to be a major mechanism for specifically killing virus-infected cells. The induction of cytotoxic T-cells which can kill such virus-infected cells is therefore an important aspect of the immune response.

CTLs recognise antigenic peptides expressed on MHC class 1 molecules of infected cells via T-cell receptors. They also express CD8, which stabilises the interaction between T-cell receptor and class 1 MHC molecules plus peptide. This cognate interaction leads to the killing of the infected cell via lytic mechanisms. Since viral surface antigens can be expressed early in the viral cycle, death of the infected cell therefore generally precedes viral replication. HIV-positive individuals who have a strong CD8+ve T-lymphocyte activity against HIV-infected cells show increased long-term survival (Hessol *et al.*, *Am. J. Epidemiol.* **30** 1167 (1989). An effective, long-lasting CTL response is therefore important both as a prophylactic and as an immunotherapeutic vaccine and a requirement of any strategy for anti-viral vaccine development. In particular a CTL response is likely to be an essential component of any therapeutic vaccine such as in HIV, HSV and HPV.

There is also evidence that CTLs can kill tumour cells both *in vivo* (de Plaen *et al.*, *Proc. Natl. Acad. Sci. USA* 1988 **85** 2274-2278) and *in vitro* - (Itoh *et al.*, *Cancer Res.* 1986 **46** 3011-3017), and induction of CTL against tumour-associated antigens may therefore have a role in cancer therapy.

CTL responses have also been detected against synthetic peptides derived from the circumsporozoite protein of *Plasmodium falciparum* (WO-A-9000402; US Department of Health) and peptides from bacteria such as *Gonococcus*, *Bordetella pertussis* (EP-A-0431237; Hoechst AG), *Listeria* or *Leishmania.*

Particulate antigen carrier systems have been developed based on the ability of the yeast retroelement Ty and the retroviral gag protein to self-assemble to form virus-like particles (VLPs) and virus-derived particles (VDPs): see WO-A-8803562 and WO-A-8803563. Such carrier systems comprise a self-assembling moiety fused to a second antigenic amino acid sequence, such as viral epitopes. One antigenic viral protein which has received much attention as a potential vaccine is the envelope glycoprotein of HIV-1, gp120. Antibodies raised against a region of this protein known as the V3 loop can neutralise viral infectivity. This region contains approximately 40 amino acids with considerable sequence variation between HIV isolates, although approximately 60% of isolates have a consensus sequence GPGRAF. Ty:VLPs incorporating the V3 region of the HIV gp120 are powerful immunogens, eliciting both humoral (antibody) and T-cell responses. For example, immunisation of rabbits with Ty VLPs carrying the V3 region from isolate IIIB (V3:Ty-VLPs) in either Freund's adjuvant or AH resulted in good antibody and neutralisation titres (Griffiths *et al*, *J. Virology* **65** 450-456 1991).

Hybrid IIIB V3:Ty-VLPs particles also cause priming of V3-specific T cells when presented either in alum adjuvant or in saline (Harris *et al.*, *Immunol.* in press (1992)). These specific T cells recognise both recombinant gp120 and V3 loop synthetic peptides of the HIV-1 IIIB isolate. Lymph node cells from C57BL/6 mice primed with IIIB V3:Ty-VLPs respond strongly to *in vitro* stimulation with synthetic peptides representing both IIIB (40 residues) and MN (39 residues) V3 loop sequences.

Hybrid VLPs derived from Ty and also hybrid VDPs derived from gag are therefore potentially useful as vaccines. They would be even more useful if presentation of the viral epitopes in this particulate system also resulted in a specific CTL response. Most methods of inducing CTL to date are based on immunisation of animals with short peptides using strong adjuvants.

CTL responses to HIV-derived proteins are of particular interest due to the urgency of developing a vaccine against this virus. CTL specific for HIV-1 gp160 (the precursor of gp120) can be primed *in vivo* in BALB/c mice using a hybrid synthetic peptide comprising the T1 gp120 T-cell epitope (Cease *et al.*, *Proc. Natl. Acad. Sci. USA* **84** 4249 (1987)) and the V3 loop B cell epitope (Palker *et al.*, *Proc. Natl. Acad. Sci. USA* **85** 1932 (1988)) using a FCA/FIA adjuvant combination (Hart *et al.*, *Proc. Natl. Acad. Sci. USA* **88** 9448 (1991). Specific CTL have also been induced by peptide fragments from gp160 (WO-A-9104045; University of Texas and WO-A-8907112; US Dept. Health). Peptides carrying the conserved epitope of HIV-1 reverse transcriptase also induce CTL when administered in adjuvant (WO-A-9113910; US Natl. Cancer Inst).

CTL responses can also be raised against peptide fragments derived from HIV core proteins p24 and p17 presented in adjuvant (WO-A-9109869 and WO-A-9101996; Medical Research Council).

Influenza virus-specific CTL against nucleoprotein peptides have been induced following immunisation of BALB/c mice with lipopeptide conjugates (Deres *et al.*, *Nature* **342** 561 (1989)) and by synthetic peptides in Freunds complete adjuvant (FCA) (Gao *et al.*, *J. Immunol.* **147** 3268 (1991)). Induction of a CTL response by peptides carrying the immunogenic determinant of haemagglutinin in FCA is also disclosed in EP-A-0366238 (SmithKline Beecham).

Lymphocytic choriomeningitis virus (LCMV)-specific CTL have been induced in BALB/c mice using a free synthetic peptide, representing a nucleoprotein T cell epitope, in Freunds Incomplete Adjuvant (FIA) (Aichele *et al.*, *J. Exp. Med.* **171** 1815 (1991)). The degree of protection against LCMV infection correlated with the ability to mount an LCMV-specific CTL response in immunised mice (Schulz *et al.*, *Proc. Natl. Acad. Sci. USA* **88** 991 (1991)). The induction of LCMV nucleoprotein specific CTL by free synthetic peptide in either FCA or FIA has also been shown to require CD4+ve T-cell help (Fayolle *et al.*, *J. Immunol.* **147** 4069 (1991)).

Protection against Sendai virus infection in B6 mice has been achieved following immunisation with a Sendai virus nucleoprotein peptide in FIA (Kast *et al.*, *Proc. Natl. Acad. Sci. USA* **88** 2283 (1991)). This nucleoprotein peptide induced high levels of Sendai virus-specific CTL, and such Sendai virus-specific CTL clones can protect lethally-infected mice (Kast *et al.*, *J. Exp. Med.* **164** 723 (1986)). The Sendai nucleoprotein peptide also induced a low level CTL response in the absence of adjuvant when injected subcutaneously but not intravenously.

The epithelial tumour antigen present in breast cancer (Hareuveni *et al.*, *Proc Natl. Acad. Sci. USA* 1990 **87** 9484-9502) and the melanoma-associated glycoprotein p97 (Estin *et al.*, *Proc Natl. Acad. Sci. USA* 1988 **85** 1052-1056) have been expressed in vaccinia. Immunisation of mice and monkeys with these constructs elicited protective humoral and cellular immune responses.

Although CTL responses have been demonstrated against a number of small peptide fragments, they are less readily induced by immunisation with exogenous whole proteins, unless the antigen is presented in a cell-associated or degraded form. Soluble proteins presented intravenously in liposomes can induce CD8+ve CTL responses (Reddy *et al., J. Immunol.* **148** 1585 (1992)). Recombinant gp160 presented in immunostimulating complexes (ISCOMs), although not in FCA, FIA or buffered saline, can induce HIV-1 envelope-specific CTL activity in BALB/c mice (Takahashi *et al.*, *Nature* **344** 873 (1990)). A specific CTL response to the V3 region of HIV envelope gp160 can also be induced by live recombinant vaccinia viruses expressing the V3 region of the IIIB, MN and RF isolates of HIV. The specificity is comparable to that induced by recombinant gp160 in ISCOMs. Cytotoxic T-cell responses have also been obtained from VSV antigens incorporated into vaccinia (US-A-4738846).

WO-A-9011085 (Molecular Biology Institute) reports induction of a CTL response in mice following immunisation with 5µm beads coated with isolated plasma membranes from tumour cells. The sheer size of the beads is likely to pose considerable difficulties in developing a human therapeutic or prophylactic agent based on such technology.

There are therefore major problems associated with all current methods of eliciting a CTL response. Firstly, although CTL responses may be induced by immunisation with certain small peptide fragments, small peptides may elicit poor antibody responses. Furthermore, in an outbred population, individuals of different MHC Class I and Class II haplotypes will respond to different peptide epitopes; many peptides may be required to prime all the possible haplotypes. However, it is by no means certain that immunisation with a large particulate antigen will produce any CTL response, since most of the available evidence indicates that immunisation with whole proteins does not give rise to a CTL response. Secondly, those CTL responses to large immunogens which have been induced require the presence of powerful adjuvants or live vectors which are not licensed for use in humans. For example, recombinant HIV gp160 presented in ISCOMs, but not in FCA, FIA or buffered saline, induced HIV-1 envelope-specific CTL activity in BALB/c mice. It is generally felt that approval for the use of ISCOMs in humans is unlikely to be forthcoming. Recombinant vaccinia viruses expressing the V3 region also induce specific CTL responses to the V3 region of HIV envelope gp160, but immunisation with vaccinia has raised concerns both about safety for use in immunosuppressed patients, and about efficacy.

There is therefore a requirement for a safe, effective way to elicit a CTL response in response to immunisation in mammals, in order to increase vaccine efficacy, both for prophylactic and immunotherapeutic purposes.

In principle, it may be thought that there are two ways in which the CTL response may be improved. First, different adjuvants from those commonly used could be sought for an improved effect: certain combinations of adjuvants may be particularly efficacious in certain circumstances;

and a number of companies are currently exploring new adjuvants, such as muramyl tripeptide. Secondly, and this is the area to which those working in the art have in practice turned in their search for enhanced CTL responses, the nature of the antigen itself can be improved.

The present invention follows neither of these avenues. It is based on the discovery and realisation that the presence of adjuvant may paradoxically suppress the CTL response, even though its presence may be appropriate or desirable for eliciting a humoral response.

According to a first aspect of the invention, there is provided a sterile, adjuvant-free pharmaceutical formulation comprising a proteinaceous particulate antigen.

Such a formulation differs from those specifically disclosed in WO-A-8803562, WO-A-8803563 and EP-A-0175261 as those publications do not specifically exemplify sterile adjuvant-free vaccine preparations. While it is true that they discuss the addition of adjuvants to vaccine formulations as an optional feature, those skilled in the art would inevitably have been induced to add an adjuvant (such as aluminium hydroxide, which is licensed for human use) as such a step would, prior to this invention, have been regarded as conventional. All presently licensed subunit vaccines (such as Hepatitis B) and killed vaccines (such as influenza) are supplied in the presence of aluminium hydroxide as an adjuvant.

The particulate antigen may comprise a plurality of self-assembling protein molecules. (The term "protein" includes glycoprotein, lipoprotein and other post-translationally or otherwise modified proteins, if the context so permits, and the term "proteinaceous" shall be construed accordingly.) The self-assembling protein molecules may be the same as or different from each other; particulate antigens which are composed of a heterologous collection of protein molecules as well as those which are composed of a homologous population of protein molecules can be used in the invention.

The self-assembling protein molecules of which the particulate antigen is formed may each comprise a first amino acid sequence which has the property of self-assembly fused to a second amino acid sequence which has the desired antigenic specificity or one of the desired antigenic specificities. The self-assembling protein molecules and particulate antigens useful in the present invention may be those specifically and generally disclosed in WO-A-8803562 and WO-A-8803563, the contents of both of which are herein incorporated by reference to the extent that the law allows. The specific examples of WO-A-8803562 and WO-A-8803563 relate to particulate antigens in which the self-assembling moiety is derived from a yeast retrotransposon-encoded (such as a yeast Ty) element; such particulate antigens are referred to as virus-like particles, or VDPs. Both WO-A-8803562 and WO-A-8803563 also disclose particulate antigens whose self-assembling moieties are encoded by viruses (particularly by retroviral *gag* genes); such particulate antigens are referred to as virus-derived particles, or VDPs. The particulate antigens will generally be less than 1$\mu$m in diameter; sizes of less than 100nm, such as in the order of 10-20nm, are typical.

The second amino acid sequence will generally include a sequence which functions as an epitope capable of inducing a cytotoxic T-lymphocyte (CTL) response in a suitable host. Such a sequence can conveniently be referred to as a CTL epitope. The CTL epitope may be part of or additional to the antigen sequence. Examples of recognised human CTL epitopes include T1, T2 and V3 loop epitopes of HIV-1 gp120 and the Th4.1 epitope of HIV-1 gp41.

Particularly preferred embodiments of the invention also include, within the second amino acid sequence a CD4 T-cell epitope, whose function is to stimulate the production of CD4 T-cells (also known as T-helper cells).

Specifically, particles for raising a CTL response may comprise a self-assembling sequence derived from the yeast retroelement Ty or the gag protein of a retrovirus, such as HIV-1, HIV-2, HTLV-I, HTLV-II, HTLV-III, SIV, BIV, ELAV, CIAV, murine leukaemia virus, Moloney murine leukaemia virus, and feline leukaemia virus. As an alternative, self assembling proteins may be derived from other, particularly viral, sources: the surface antigen of hepatitis B is an example and is the subject of EP-A-0175261. Other examples include Hepatitis B core antigen, bluetongue virus, human papilloma virus and herpes simplex virus I and II and HSV-6. The antigenic sequence may correspond to a sequence derived from or associated with an aetiological agent or a tumour. The aetiological agent may be a microorganism such as a virus, bacterium, fungus or parasite. The virus may be: a retrovirus, such as HIV-1, HIV-2, HTLV-I, HTLV-II, HTLV-III, SIV, BIV, ELAV, CIAV, murine leukaemia virus, Moloney murine leukaemia virus, and feline leukaemia virus; an orthomyxovirus, such as influenza A or B; a paramyxovirus, such as parainfluenza virus, mumps, measles, RSV and Sendai virus; a papovavirus, such as HPV; an arenavirus, such as LCMV of humans or mice; a hepadnavirus, such as Hepatitis B virus; a herpes virus, such as HSV, VZV, CMV, or EBV. The tumour-associated or derived antigen may for example be a proteinaceous human tumour antigen, such as a melanoma-associated antigen, or an epithelial-tumour associated antigen such as from breast or colon carcinoma.

The antigenic sequence may be derived from a parasite, such as *Plasmodium falciparum,* or a bacterium, such as of genus *Neisseria*, *Bordetella*, *Listeria*, *Mycobacteria* or *Leishmania*.

Preferred self-assembling sequences are derived from the yeast retroelement Ty or the gag protein of a retrovirus, particularly HIV-1.

Particularly preferred second amino acid sequences are antigenic sequences corresponding to a viral or tumour antigen CTL epitopes from a retrovirus, a paramyxovirus, an arenavirus or a hepadna virus, or a human tumour cell. Examples include epitopes from:

1) HIV (particularly HIV-1) gp120,
2) HIV (particularly HIV-1) p24,
3) Influenza virus nucleoprotein,
4) LCMV nucleoprotein,
5) HPV L1 and L2 proteins,
6) p97 melanoma associated antigen,
7) GA 733-2 epithelial tumour-associated antigen,
8) MUC-1 repeat sequence from epithelial tumour-associated antigen,
9) mycobacterium p6,
10) malaria CSP or RESA antigens, and
11) human papilloma virus E5 and E7.

Other components, particularly lipid, which contribute to antigenicity may be present. Viral-derived particles will generally be free of infectious nucleic acid.

The invention can be useful in eliciting a CTL response to a number of different antigens, such as those of variants of the viral epitope region, by immunisation with formulations of the invention consisting of a plurality of different fusion proteins. Preferred are particles where the self-assembling moiety of Ty or HIV-1 gag is fused to a series of HIV V3 regions.

A sequence which is said to have the function of or be derived from a natural sequence may be, and preferably in some cases is, identical to a natural sequence. However, it will be appreciated that sequences which are not identical to natural sequences may work perfectly adequately and may even in some cases be better; for this reason their use is not ruled out. The invention therefore encompasses sequences which have qualitatively the same (relevant) function as a natural sequence but which have different amino acid or nucleotide base compositions. There will usually be substantial homology between natural sequences and other sequences having qualitatively the same function; this homology may be at least 50%, 60%, 70% or 80%, or even at least 90%, 95% or 99%, in increasing order of preference.

Alternatively or in addition, nucleotide sequences useful in the invention may hybridise to a natural sequence whose function is sought, or would do so but for the degeneracy of the genetic code. Hybridisation may be under stringent conditions (see Maniatis *et al.*, "Molecular Cloning: A Laboratory Manual", Cold Spring Harbor Laboratory (1982), pp 387-389). An example of stringent hybridisation conditions is hybridisation at 4xSSC at 65°C, followed by washing in 0.1xSSC at 65°C for one hour. An alternative exemplary hybridisation condition is 50% formamide, 4xSSC at 42°C.

If hybridisation is not under stringent conditions, it may be under relaxed conditions. Examples of such non-stringent hybridisation conditions are 4xSSC at 50°C or hybridisation with 30-40% formamide at 42°C.

Formulations of the invention are useful as prophylactic or, particularly, immunotherapeutic vaccines. A vaccine may comprise the particulate antigen in a physiologically acceptable non-toxic carrier. Sterility will generally be essential for parenterally administrable vaccines. Preferred physiologically acceptable non-toxic carriers are sterile physiological saline or sterile PBS.

Vaccines in accordance with the invention may present more than one antigen. Either a cocktail of different antigens may be used, or a homogenous population of particulate antigens having more than one epitope could be used, as described above. It may in practice be simpler for a vaccine to contain a mixture of different particulate antigens.

Formulations in accordance with the first aspect of the invention are sterile. Sterility may be achieved by any convenient way. Filter sterilisation, for example through a 0.22μm filter, is particularly suitable.

According to a second aspect of the invention, there is provided the use of a proteinaceous particulate antigen in the preparation of an adjuvant-free pharmaceutical immunotherapeutic or prophylactic vaccine formulation for inducing a cytotoxic T cell response.

It will be appreciated that the invention will be useful in a method of inducing a cytotoxic T cell response, the method comprising administering to a subject an adjuvant-free formulation comprising a proteinaceous particulate antigen. The dosage regimen will be under the guidance of the physician or clinician and will generally be such as to be effective but non-toxic. The formulation may be administered either as a single dose or as a number of doses. Repeat dosage regimens may be administered over a period of days, weeks, months or years. However, for CTL responses, a single dose is preferred.

The invention finds particular application in stimulating a full humoral (antibody) and cell-mediated (CTL and preferably T-helper cell) immune response to a non-infectious antigen. This can be achieved by a combination strategy comprising administering non-adjuvanted immunogen (in the form of a non-adjuvanted formulation, for example in accordance with the first aspect of the invention), to prime CD8 CTL, CD4 T cell and B cell responses, and also administering adjuvanted immunogen to boost the CD4+ve T cell and B cell compartment. Examples of suitable adjuvants include muramyl peptide compounds such as prototype muramyl dipeptide, aluminium hydroxide, saponin and liposomes.

According to a third aspect of the invention, therefore, there is provided an immunotherapeutic or prophylactic vaccination kit comprising a first, non-adjuvanted, proteinaceous particulate antigen formulation and a second adjuvanted proteinaceous particulate antigen formulation. The first and second formulations will generally be sterile.

According to a fourth aspect of the invention, there is provided a product comprising first and second formulations as described above for combined, simultaneous or sequential administration in the treatment or prophylaxis of disease amenable to immunotherapy or prophylaxis, particularly viral or other microbial infection or cancer.

The invention is useful in a method of immunotherapy or prophylaxis, the method comprising administering to a subject an effective amount of an adjuvant-free formulation in accordance with the first aspect of the invention (to stimulate a CTL response) and an adjuvanted formulation (to stimulate a humoral response). Again, the dosage regimen will be under the guidance of the physician or clinician and will generally be such as to be effective but non-toxic.

Preferred features of each aspect of the invention are as for the first aspect, *mutatis mutandis*.

The following examples illustrate the invention, but are not intended to limit the scope in any way. The examples refer to the accompanying drawings, in which:

FIGURES 1A, 1B and 1C show *in vivo* CTL priming by hybrid V3:Ty-VLPs (Figure 1A relates to Example 1 and Figures 1B and 1C relate to Example 2);

FIGURES 2A and 2B show rapid induction of potent V3-specific CTL responses by hybrid V3:Ty-VLPs (Figure 2A relates to Example 3 and Figure 2B to Example 4);

FIGURE 3 relates to Example 5 and shows MN V3-specific CTL responses following immunisation with MN V3:Ty-VLPs;

FIGURE 4 relates to Example 6 and shows that hybrid V3:Ty-VLPs are superior to V3 peptide and gp120 in inducing V3-specific CTL responses; and

FIGURE 5 relates to Example 7 and shows V3-specific CTL responses following immunisation of BALB/c mice with GAG-V3 particles with either Freund's complete adjuvant or aluminium hydroxide adjuvant or, alternatively, without adjuvant.

## EXAMPLE 1

### *In vivo* CTL priming by hybrid V3:Ty-VLPs without adjuvant

The immunogenicity of hybrid V3:Ty-VLPs was examined. Hybrid V3:Ty VLPs were produced essentially according to the method of Example 3 of WO-A-8803562. Synthetic encoding gp160 amino acids 295-334 (Los Alamos database, IIIB HXB2 V3 loop) in the *Bam*HI site of plasmid pOGS40 which contains a truncated TYA gene encoding amino acids 1-381 of protein p1. (pOGS40 is a derivative of pMA5620 which is described in WO-A-8803562: The PGK promoter of pMA5620 was replaced by a hybrid promoter comprising the 5' non-coding region of the PGK gene and the upstream activation sequence of the GAL110 promoter (EP-A-0258067)). The resulting plasmids were transformed into yeast cells as described in WO-A-8803562 and the V3:Ty fusion proteins expressed at high levels. The self-assembling V3:Ty-VLPs were purified on a sucrose density gradient followed by size exclusion chromatography.

BALB/c mice (H-$2^d$) were immunised s.c. with IIIB V3:Ty-VLPs either in FCA, alum or in saline; the vaccines without adjuvant were filter sterilisable through a 0.22μm filter prior to administration. Following a repeat dose on day 46, splenocytes were removed and restimulated *in vitro* with 40mer IIIB V3 loop peptide and 2% Rat ConA supernatant (RCAS) for seven days prior to testing against $^{51}$chromium-labelled P815 target cells (H-$2^d$) incubated with the 40mer IIIB V3 peptide (p815 cells express no MHC class II and will therefore only present the peptide in conjunction with MHC class I. CTL will recognise certain peptide sequences, but only in conjunction with MHC class I). High levels of IIIB V3-specific CTL were induced by IIIB V3:Ty-VLPs when injected without adjuvant (Figure 1A). Low levels of CTL activity were detected at the highest E:T ratio (100:1) in effector cells from mice immunised with IIIB V3:Ty-VLPs in FCA. No significant CTL priming was induced using alum adjuvant. There was no (<5.0%) killing of control P815 cells in the absence of peptide sensitisation at any E:T ratio in the three groups of effector cells.

Referring in more detail to Figure 1A, BALB/c mice were immunised s.c. with 200μg of IIIB V3:Ty-VLPs either in alum (●), FCA (O) or with no adjuvant (■) on days 0 and 46. On day 70, spleens were removed from two mice per group and single cell suspensions prepared and pooled. The pooled spleen cells were restimulated *in vitro* for 7 days in RPMI1640 culture medium (10ml) containing 10% foetal calf serum (TCM-FCS) and 5µg/ml of 40 mer IIIB V3 peptide (CRB Ltd). After 24 hours, ammonium sulphate-concentrated supernatant from ConA-stimulated rat spleen cells (29) was added (2% final volume) as a source of IL-2 and other cytokines. After 7 days, the effector (E) cells were then washed once and resuspended in TCM-FCS and 100µl aliquots were added to 100µl of target (T) cells (1 x $10^4$/well) at various E:T ratios in triplicate or quadruplicate wells of U-bottomed 96 well mictrotitre plates. Target cells were P815 (H-$2^d$) cells (1 x $10^6$/ml) incubated overnight at 37°C with 20µg/ml of 40 mer IIIB V3 peptide. Peptide-pulsed and control P815 cells were labelled with $^{51}$Cr (1 hour at 37°C, 20µCi/$10^6$ cells) then washed before adding to the effectors. After 4-5 hours, 100µl of supernatant was removed from each well for counting. The % specific lysis was calculated as 100 x [(test counts - spontaneous counts)/-(maximum counts - spontaneous counts)]. Maximum release was generated by adding 100µl of 5% Triton-X 100 to 100µl of target cells. The background specific lysis of control target cells in the presence of V3:Ty-VLP primed effector cells did not exceed 5% at any E:T ratio. Spontaneous release from target cells were 11% (P815 cells + peptide) and 10.1% (P815 cells, no peptide). Error bars shown standard error from the mean.

## EXAMPLE 2

### Specificity of CTL induced by hybrid V3:Ty-VLPs

Spleen cells from mice immunised with IIIB V3:Ty-VLPs without adjuvant, as described in Example 1 above, were restimulated *in vitro* with the 40mer IIIB V3 peptide and the effector cells generated were tested against P815 target cells incubated with either the 40mer IIIB V3 loop peptide or a 15 mer IIIB V3 peptide representing the central portion of the V3 loop (Table 1). P815 cells were recognised as target cells by the V3-specific CTL when preincubated with the 40mer V3 loop peptide or the V3 loop tip peptide (Figure 1B). The effector cells primed by IIIB V3: Ty-VLPs were isolate-specific and did not recognise P815 cells incubated with peptides representing the V3 loop sequences of MN or RF isolates (specific lysis <5% at 100:1 E:T ratio). There was no specific killing if P815 (H-$2^d$) cells were replaced by EL-4 (H-$2^b$) target cells (data not shown). When the E:T ratio was held constant and the long and short IIIB V3 peptides titrated, there was a 2-log difference in the concentration required to give 50% lysis (Figure 1C). These data indicate that the specificity of the CTL induced by IIIB V3:Ty-VLPs is similar to those induced by recombinant vaccinia expressing gp 160 and recombinant gp 160 in ISCOMs (24,17). The results also indicate that the 15 mer V3 peptide can be presented by MHC class I molecules on the P815 cells far more efficiently than the 40mer V3 peptide. It is likely that the larger peptide may require internalisation and processing by P815 cells or extracellular degradation prior to being presented at an optimal length with class I molecules. Exogenous soluble protein antigens are known not to be processed by P815 cells for CTL recognition (Carbone and Bevan, *J. Exp. Med*. **171** 377 (1990)) however little is documented on the processing of small polypeptides.

Referring in more detail to Figure 1B, pooled spleen cells from two mice immunised as in relation to Figure 1A (Example 1) above were prepared on day 62 and restimulated for 9 days *in vitro* as above with 40 mer IIIB V3 peptide. Effector cells were assayed for CTL activity against P815 cells pre-incubated as above with 20µg/ml of 40 mer IIIB V3 peptide (O), 15 mer IIIB V3 loop tip peptide 734 (■) or no peptide (□). Spontaneous release values were 15.9% (P815 cells + 40 mer V3 peptide), 13.6% (P815 cells + 15 mer V3 loop tip peptide) and 16.3% (P815 cells, no peptide).

Referring in more detail to Figure 1C, CTL assay using effector cells described in relation to Figure 1A (Example 1) and P815 target cells with either the 40 mer IIIB V3 loop or 15 mer IIIB V3 loop tip peptides added directly to the assay at various concentrations. The E:T ratio was 50:1. Spontaneous release from P815 cells was 11.9%.

## EXAMPLE 3

### Potency of V3:Ty-VLPs in inducing CD8+ve, H-$2^d$-restricted V3-specific CTL responses

The dose response effect of IIIB V3:Ty-VLPs injected once i.m. into BALB/c mice without adjuvant was examined. Spleens were removed at a much earlier time point (day 18) than in the previous experiments. Substantial CTL priming was induced at all four doses given (Figure 2A). A peak of CTL activity was observed in effector cells generated from mice immunised with a 20µg dose (50% lysis at E:T ratio of 10:1). A 5µg dose induced a superior CTL response (50% lysis at 27:1) than the 100µg (50% lysis at 87:1) and 50µg (50% lysis at 49:1) doses indicating that too much immunogen may have a deleterious effect on CTL priming at this time point by this route. These data demonstrate that the hybrid V3:Ty-VLPs are potent CTL immunogens leading to rapid and substantial CTL responses. When anti-CD4 (GK1-5) and anti-CD8 (53-6-72) rat monoclonal antibodies (Mabs) were added directly to the CTL assay, selective inhibition of CTL activity was observed only in cultures treated with the anti-CD8 Mab (29.0 and 36.7% inhibition at 50:1 and 25:1 E:T ratios, respectively) demonstrating that the effector cells are CD8+ve. Furthermore, immunisation of C57BL/6 (H-$2^b$) mice with 100µg of IIIB V3:Ty-VLP did not induce CTL priming (using EL-4 cells as targets, data not shown) which indicates that the IIIB V3-specific CTL are restricted at the H-$2^d$ loci.

Referring in more detail to Figure 2A, BALB/c mice were immunised i.m. with 100 (O), 50 (●), 20 (□) or 5µg (■) of IIIB V3:Ty-VLPs in saline. On day 18, pooled splenocytes (2 spleens per group) were restimulated for 6 days with 40 mer V3 peptide and effector cells were tested for lysis of 40 mer V3 peptide-pulsed or control P815 target cells as described in Fig. 1A. Control target cell lysis was <5.0% at all E:T ratios for all doses. Spontaneous release was 11.5% (P815 cells + V3 peptide) and 13.5% (P815 cells, no peptide). Standard error of the replicates was <5%.

EXAMPLE 4

*In vitro* processing of IIIB V3:Ty-VLPs

To confirm that IIIB V3:Ty-VLPs can gain access to the correct antigen processing pathway for MHC class I presentation, P815 cells were pulsed overnight with either IIIB V3:Ty-VLPs, 40mer IIIB V3 peptide, or non-hybrid control Ty-VLPs. These target cells and control P815 cells were then labelled with $^{51}Cr$ and tested against IIIB V3:Ty-VLP-induced effector CTL. Both the V3 peptide- and IIIB V3:Ty-VLP-pulsed target cells were effectively killed by the V3-specific CTL (Figure 2B) indicating that P815 cells can process particulate immunogens and re-express CTL epitopes on MHC class I molecules. These cells have previously been shown not to process soluble proteins for class I presentation (Carbone and Bevan, *J. Exp. Med*. **171** 377 (1990)).

Referring in more detail to Figure 2B, effector cells from the 100µg dose group described above were tested against P815 cells pulsed overnight with either 40 mer V3 peptide (50µg/5 x $10^5$ cells), IIIB V3:Ty-VLPs (100µg/5 x $10^5$ cells), control Ty:VLPs (100µg/5 x $10^5$ cells) or no antigen. The E:T ratio was 60:1.

EXAMPLE 5

Specificity of V3-specific CTL induced by MN V3:Ty-VLPs

Previous data have indicated that the MN V3 loop contains a CTL epitope which is restricted by H-$2D^d$. BALB/c mice were immunised i.m. with 100µg hybrid MN V3:Ty-VLPs to investigate if these would induce MN V3-specific CTL responses. Substantial levels of CTL activity were detected against P815 cells pulsed with MN but not IIIB or RF peptides (Figure 3). There was a slight increase in the killing of IIIB V3 peptide-pulsed P815 cells above that observed with the RF V3 peptide and non-pulsed P815 cells and this may reflect low level cross-reactivity. Therefore, hybrid V3:Ty-VLP immunogens appear to be capable of priming CTL of similar specificity to those induced by live vaccinia-gp 160 and ISCOM-adjuvanted recombinant gp 160 without the requirement for adjuvant and at relatively low doses.

Figure 3 shows MN V3-specific CTL responses following immunisation with MN V3:Ty-VLPs. BALB/c mice were immunised i.m. with 100µg of hybrid VLPs. On day 18, spleens were removed and pooled splenocytes (2 spleens) were restimulated for 6 days with MN V3 peptide (5µg/ml). Effector cells were assayed against P815 target cells pulsed overnight with 20µg/ml of either the IIIB (■), MN (□) or RF (●) V3 loop peptides or no peptide (O). Spontaneous release was 17.6% (P815 + IIIB V3 peptide) 18.7% (P815 + MN V3 peptide) 19.8% (P815 + RF V3 peptide) 17.7% (P815 cells, no peptide).

EXAMPLE 6

Comparative immunogenicity of V3:Ty-VLPs, recombinant gp 120 and V3 synthetic peptide

To compare the immunogenicity, in terms of CTL priming, of hybrid V3:Ty-VLPs with synthetic peptide (40mer IIIB V3 loop) and recombinant gp 120 without adjuvant, BALB/c mice were immunised with the three types of V3 loop immunogen at equivalent V3 loop dose (2µg/mouse) and also with a much higher dose of V3 peptide (100µg dose). Effector cells from mice immunised with IIIB V3:Ty-VLPs i.m. gave 50% killing at an E:T ratio of approximately 4:1, whilst the s.c. route was less efficient (50% lysis at 10:1). The gp 120-induced effector cells were approximately 25-fold less potent than those induced by IIIB V3:Ty-VLPs and the V3 peptide-induced effectors only produced 10.3% killing at 100:1 ratio even at 100µg/mouse (Figure 4).

*In vitro* re-stimulation of normal spleen cells with the V3 peptide did not induce V3-specific CTL confirming that the CTL we are detected in this system is a result of *in vivo* priming. Interestingly, V3-specific CTL in fresh, non-restimulated splenocytes from mice immunised with V3:Ty-VLPs has so far not been detected. This would indicate that *in vivo* priming leads to the proliferation of CD8+ve precursor CTL which require additional stimuli (differentiation) before they are capable of killing appropriate target cells and further *in vivo* boosting does not appear to provide these stimuli.

Figure 4 shows that hybrid V3:Ty-VLPs are superior to V3 peptide and gp120 in inducing V3-specific CTL responses. BALB/c mice were immunised i.m. with either 40 mer IIIB V3 peptide at 100 (□) or 2μg (●), recombinant gp120 (affinity-purified rgp120, CHO-derived, MRC-ADP) at 23μg (O), IIIB V3:Ty-VLPs at 20μg (●), IIIB V3:Ty-VLPs 20μg injected s.c. (△) or non-immunised mice (▲). On day 25, pooled splenocytes (2 spleens per group) were restimulated for 6 days with IIIB V3 peptide and effector cells assayed as described in Fig. 1A. Spontaneous release was 12.3% (P815 + V3 peptide) and 12.4% (P815 cells, no peptide). % specific lysis values of control P815 target cells at each E:T ratio were subtracted from the peptide pulsed P815 cell lysis values to give net % specific lysis. Control P815 target cell lysis at 100:1 E:T ratio was 21.1% (rgp 120), 8.8% (V3 peptide, 2μg), 13.9% (V3 peptide, 100μg) 0% (IIIB V3:Ty-VLP, i.m.) 7.7% (IIIB V3:Ty-VLP, s.c.) and 0% (non-immune). Standard error of the replicates as <5%.

EXAMPLE 7

Specificity of CTL induced by hybrid gag:V3-VDPs

The preparation of hybrid particles formed by a truncated gag moiety fused to a V3 peptide and was undertaken as follows:

HIV-1 *gag* constructs were derived from plasmid pOGS2. pOGS2 contains nucleotides 678-2470 of HIV-1 BH10 (Ratner *et al* 1985 *Nature* **313**, 277) inserted into plasmid pSP64 (Melton *et al* 1984 *Nucl. Acids Res.* **12**, 7035) as an *Sst*I-*Bcl*I restriction fragment. The *gag* gene is encoded by nucleotides 790-2325. In order to provide the *gag* gene on a convenient *Bam*HI fragment, pOGS2 was modified using *Bal*31 exonuclease and *Bam*HI linkers. To modify the 3' end of the *gag* fragment, plasmid pOGS2 was cleaved with *Sal*I, digested with *Bal*31 exonuclease for various times and re-ligated in the presence of excess *Bam*HI linkers (CCGGATCCGG). The deletion end points of the resulting plasmids were determined by DNA sequencing. Plasmid pOGS11 is a deletion derivative in which the *Bam*HI site is at position +49 with respect to the *gag* termination codon. To modify the 5' end of the *gag* fragment, pOGS2 was cleaved with *Eco*R1, digested with *Bal*31 exonuclease for various times and re-ligated in the presence of excess *Bam*HI linkers (CCGGATCCGG). The deletion endpoints were determined by DNA sequencing. Plasmid pOGS12 is a deletion derivative in which the *Bam*HI site is at position -22 with respect to the *gag* initiating ATG codon. In order to provide a *gag* gene flanked by *Bam*HI sites, the 3' end of the *gag* gene in pOGS12 was replaced with a *Bgl*II-*Bgl*I fragment from pOGS11 containing the modified sequence. The resulting plasmid was designated pOGS15.

In order to produce a *gag* gene in which the C-terminal 75 residues have been removed, synthetic oligomers SEAGAG/08 and SEAGAG/09 were inserted into pOGS554 which had been cleaved with *Bgl*II. This insertion results in the generation of a stop codon and a *Bam*HI site downstream of the *Bgl*II site. The resulting plasmid was designated pOGS562 and contains a truncated *gag* gene as a *Bam*HI cassette.

A fusion gene containing the truncated HIV-1 *gag* gene and a V3 loop sequence was made by inserting synthetic oligomers encoding the V3 loop sequence from HIV-1 isolate HXB2 (Fisher *et al* 1986 *op cit*) at the *Bgl*II site in plasmid pOGS562. The resulting plasmid was designated pOGS563 and contains the *gag*(truncated):V3 loop fusion gene as a *Bam*HI cassette.

The truncated *gag*/V3 loop cassette was excised from pOGS563 as a *Bam*HI fragment and cloned into the baculovirus expression vector pAcRP23 to generate pOGS574. Plasmid pAcRP23 contains the powerful polyhedrin promoter from *Autographa californica* nuclear polyhedrosis virus (AcNPV) and a unique *Bam*HI site for insertion of foreign genes flanked by AcNPV sequences. Plasmid pAcRP23 also contains sequences for replication and selection of the plasmid in *E. coli* - (Matsuura *et al* 1987 *J. Gen. Virol* **68** 1233). When cloned into the *Bam*HI site of this vector, the *gag* gene derivatives come under the control of the polyhedrin promoter.

The cell line designated *Spodoptera frugiperda* 9 (SF9) (Summers and Smith 1987 *Texas Agricultural Experiment Station* Bulletin No. 1555), which is a clonal derivative of the lepidopteran line IPLB-Sf21-AE (Vaughn *et al* 1977 *In vitro* **13** 213), was used. Cells were grown in suspension culture in medium comprising TC100 (GIBCO-BRL, UK) containing 10% heat inactivated foetal calf serum (ICN-FLOW Laboratories, UK) plus 501U/ml penicillin and 50mg/ml streptomycin (GIBCO-SRL, UK).

Recombinant baculoviruses were prepared using the phenomenon of *in vivo* recombination between homologous DNA sequences. The viral (AcNPV) DNA and plasmid DNA (pOGS553, pOGS556, pOGS572 or pOGS574) were introduced into SF9 insect cells by the method of calcium chloride precipitation and progeny were recovered after 48 hours (Summers and Smith, *op cit*). Recombinant baculoviruses were selected on the basis of a polyhedrin-negative phenotype in a Brown & Faulkner plaque assay (Brown and Faulkner 1977 *J. Gen. Virol* **36** 361). After plaque purification, high titre viral stocks of the recombinant viruses were prepared and their titres determined in plaque assays.

The cells were harvested at 48 hours post-infection and pelleted by centrifugation at 1000g. After washing with phosphate buffered saline (PBS), the cells were lysed and the particles harvested according to standard procedures.

CTL responses were measured following immunisation with GAG:V3 particles. Mice received a single intramuscular dose of 20µg either in FCA, with an aluminium hydroxide adjuvant or without adjuvant. Formulations without adjuvant were filter sterilisable through a 0.22µm filter prior to administration. Splenocytes were removed after 28 days and expanded by clonal proliferation *in vitro* for 6 days with a 40 mer IIIB V3 peptide corresponding to the sequence of the IIIB V3 loop. The effector cells induced in this way were tested for their cytotoxic activity against control p815 cells or p815 cells labelled with IIIB V3 40 mer peptide.

A high level of specific lysis of IIIB peptide-labelled target cells was observed after 6 days using effector cells from the mice immunised without adjuvant, and this titrated out to low E:T ratios (Figure 5). A low level of specific lysis was seen using effector cells from mice immunised with FCA.

## EXAMPLE 8

### Induction of CTL responses by particulate Hepatitis B surface antigen

The hepatitis B surface antigen has previously been shown to be capable of assembly into particles (EP-A-0175261).

This protein contains a number of CTL epitopes (Moriyama *et al*, (1990), *Science* **248** 361-4). CTL responses can therefore be measured following immunisation of mice with Hepatitis B surface antigen particles obtainable from Bionike, CA, USA. Mice receive a single sterile intramuscular dose in the range of 5-100µg in the absence of adjuvant. Splenocytes are removed after 20 days and expanded by clonal proliferation *in vitro* for 7 to 12 days with hepatitis B surface antigen. The effector cells induced in this way are incubated with cells of a cognate haplotype which are control cells or cells labelled with Hepatitis B surface antigen, resulting in the lysis of those target cells where the MHC class I antigen bears peptides corresponding to the CTL epitope.

## EXAMPLE 9

### Induction of CTL responses by particulate Hepatitis B core antigen

A number of CTL epitopes have been described within the hepatitis B core antigen, e.g. Ehata *et al*, (1992), *J. Clin. Invest.* **89** 332-8; Guilhot *et al*, (1992), *J. Virol.* **66** 2670-8; Pena *et al*, (1991), *J. Exp. Med.* **174** 1565-70. Mice receive a single sterile intramuscular dose in the range of 5-100µg Hepatitis B core antigen particles in the absence of adjuvant. Splenocytes are removed after 20 days and expanded by clonal proliferation *in vitro* for 7 to 12 days with hepatitis B core antigen. The effector cells induced in this way are incubated with cells of a cognate haplotype which are control cells or cells labelled with hepatitis B core antigen, resulting in the lysis of those target cells where the MHC class I antigen bears peptides corresponding to the CTL epitope.

## EXAMPLE 10

### Induction of CTL responses by hybrid Hepatitis B surface antigen particles

The hepatitis B surface antigen has previously been shown to be capable of assembly into hybrid particles which contain a particle-forming sequence from the hepatitis B surface antigen and a second biologically active sequence (EP-A-0175261). Such second heterologous sequences may contain a CTL epitope. Hybrid particles comprising the particle forming sequence from the hepatitis B surface antigen and the V3 loop sequence of the IIIB variant of HIV-1 fused at the N-terminus of the hepatitis B surface antigen as described in EP-A-0175261 are used to measure CTL responses in BALB/c mice. Mice receive a single sterile intramuscular dose in the range of 5-100µg in the absence of adjuvant. Splenocytes are removed after 20 days and expanded by clonal proliferation *in vitro* for 7 to 12 days with a 40mer or smaller IIIB V3 peptide corresponding to the sequence of the IIIB V3 loop. The effector cells induced in this way are incubated with control p815 cells or p815 cells labelled with the 40mer IIIB V3 peptide, resulting in the lysis of those target cells where the MHC class I antigen bears peptides corresponding to the CTL epitope.

EXAMPLE 11

Induction of CTL responses by hybrid Hepatitis B core:V3 antigen particles

Hybrid particles which contain a particle-forming sequence from the hepatitis B core antigen and a second sequence containing a CTL epitope from the V3 loop sequence of the IIIB variant of HIV-1 are used to measure CTL responses in BALB/c mice. Mice receive a single sterile intramuscular dose in the range of 5-100μg in the absence of adjuvant. Splenocytes are removed after 20 days and expanded by clonal proliferation *in vitro* for 7 to 12 days with a 40mer or smaller IIIB V3 peptide corresponding to the sequence of the IIIB V3 loop. The effector cells induced in this way are incubated with control p815 cells or p815 cells labelled with the 40mer IIIB V3 peptide, resulting in the lysis of those target cells where the MHC class I antigen bears peptides corresponding to the CTL epitope.

EXAMPLE 12

Induction of CTL responses by single-shelled particulate bluetongue virus

The VP3 and VP7 core proteins of bluetongue virus (BTV) are together capable of forming empty BTV core-like particles (French, T.J. and Roy, P., (1990), *J. Virol*. **64** 1530-1536). CTL responses are measured following immunisation of mice with such bluetongue virus particles. Mice receive a single sterile intramuscular dose in the range of 5-100μg in the absence of adjuvant. Splenocytes are removed after 20 days and expanded by clonal proliferation *in vitro* for 7 to 12 days with VP3/VP7 particles. The effector cells induced in this way are incubated with cells of a cognate haplotype which are control cells or cells labelled with VP3/VP7 particles, resulting in the lysis of those target cells where the MHC class I antigen bears peptides corresponding to the CTL epitope.

EXAMPLE 13

Induction of CTL responses by double-shelled particulate bluetongue virus

The VP3 and VP7 core proteins of bluetongue virus are capable of forming non-infectious, double shelled proteinaceous particles when expressed with the BTV outer capsid proteins VP2 and VP5 (French, T.J., Marshall, J.J.A. and Roy, P., (1990), *J. Virol*., **64** 5695-5700). CTL responses are measured following immunisation of mice with such double-shelled bluetongue virus particles. Mice receive a single sterile intramuscular dose in the range of 5-100μg in the absence of adjuvant. Splenocytes are removed after 20 days and expanded by clonal proliferation *in vitro* for 7 to 12 days with double-shelled bluetongue virus particles. The effector cells induced in this way are incubated with cells of a cognate haplotype which are control cells or cells labelled with double-shelled bluetongue virus particles, resulting in the lysis of those target cells where the MHC class I antigen bears peptides corresponding to the CTL epitope.

EXAMPLE 14

Induction of CTL responses by hybrid single-shelled particulate bluetongue virus

The VP3 and VP7 proteins of bluetongue virus are capable of assembly into hybrid particles which contain a heterologous biologically active sequence fused at the N-terminus of VP7. Such sequences may contain a CTL epitope. Hybrid particles comprising the VP3 and VP7 core proteins of bluetongue virus and the V3 loop sequence of the IIIB variant of HIV-1 are used to measure CTL responses in BALB/c mice. Mice receive a single sterile intramuscular dose in the range of 5-100μg in the absence of adjuvant. Splenocytes are removed after 20 days and expanded by clonal proliferation *in vitro* for 7 to 12 days with a 40mer or smaller IIIB V3 peptide corresponding to the sequence of the IIIB V3 loop. The effector cells induced in this way are incubated with control p815 cells or p815 cells labelled with the 40mer IIIB V3 peptide, resulting in the lysis of those target cells where the MHC class I antigen bears peptides corresponding to the CTL epitope.

EXAMPLE 15

Induction of CTL responses by hybrid double-shelled particulate bluetongue virus

The VP3, VP7, VP2 and VP5 proteins of bluetongue virus are capable of assembly into hybrid particles which contain a heterologous, biologically active sequence fused at the N-terminus of VP7. Such heterologous sequences may contain a CTL epitope. Hybrid particles comprising the VP3, VP7, VP2 and VP5 proteins of bluetongue virus and the V3 loop sequence of the IIIB variant of HIV-1 are used to measure CTL responses in BALB/c mice. Mice receive a single sterile intramuscular dose int he range of 5-100μg in the absence of adjuvant. Splenocytes are removed after 20 days and expanded by clonal proliferation *in vitro* for 7 to 12 days with a 40mer or smaller IIIB V3 peptide corresponding to the sequence of the IIIB V3 loop. The effector cells labelled with the 40mer IIIB V3 peptide, resulting in the lysis of those target cells where the MHC class I antigen bears peptides corresponding to the CTL epitope.

EXAMPLE 16

Induction of CTL responses by Human Papillomavirus-16 L2 VLPs

Human papillomavirus-L2 (minor capsid) protein (HPV-L2) is thought to contain a number of CTL epitopes. Hybrid particles of HPV-L2 fused to Ty (HPV- L2:Ty VLPs) were prepared by constructing a vector, pOGS 348, as follows. A fusion gene containing the Ty gene and the human papillomavirus-L2 gene was made by inserting a 300 base pair sequence corresponding to base nos. 4568-4865 of the L2 gene into the *Bam*HI cloning site of pOGS 40. The resulting plasmids were transformed into yeast strains as described in WO-A-8803562. The HPV-L2:Ty VLPs were expressed at high levels, purified by sucrose density gradient and size exclusion chromatography and analysed by SDS/PAGE. Hybrid particles were sterilised and 20μg used to immunise mice by a single intramuscular dose in the absence of adjuvant. Three strains of mice were used for immunisation; Balb/c ($H2^d$), C3H/Hej($H2^k$) and C57BL/6 ($H2^b$). Splenocytes are removed after 20 days and expanded by clonal proliferation *in vitro* for 7 to 12 days with hybrid HPV-L2:Ty VLPs. The effector cells induced in this way are incubated with target cells of a cognate haplotype which are either control cells or cells labelled with HPV-L2:Ty VLPs, resulting in the lysis of those target cells where the MHC class I antigen bears peptides corresponding to the CTL epitope. Cells of a cognate haplotype for Balb/c mice are p815 cells; those for C3H mice are LBRM-33-IAS cells; and those for C57 mice are EL4 cells.

EXAMPLE 17

Induction of CTL responses by V3:Ty VLPs in macaques

IIIB and MN V3:Ty VLPs were prepared as described above. Macaques were immunised with at least one sterile intramuscular dose of 200μg in the absence of adjuvant. CTL activity was assessed after immunisation by expanding peripheral blood lymphocytes (PBL) *in vitro* for 7 to 12 days with 40mer or smaller V3 peptides to create effector cells. PBLs were also stimulated with PHA or ConA and labelled with 40mer or smaller V3 peptides to provide target cells. Incubation of target and effector cells results in the lysis of those target cells where the MHC class I antigen bears peptides corresponding to the CTL epitope.

EXAMPLE 18

Induction of CTL responses by V3:gag VDPs in macaques

IIIB and MN V3:gag VLPs were prepared as described above. Macaques were immunised with at least one sterile intramuscular dose of 200μg in the absence of adjuvant. CTL activity was assessed after immunisation by expanding peripheral blood lymphocytes (PBL) *in vitro* for 7 to 12 days either with 40mer or smaller V3 peptides or with gagVDPs to create effector cells. PHBLs were also stimulated with PHA or ConA and labelled with gag-VDPs, overlapping gag peptides or 40mer or small V3 peptides to provide target cells. Incubation of target and effector cells results in the lysis of those target cells where the MHC class I antigen bears peptides corresponding to the CTL epitope.

EXAMPLE 19

Induction of CTL responses by p24-VLPs in macaques

The HIV p24 protein has been shown to contain an epitope for CTLs (Nixon, D.F. *et al*, *Nature* (1988) **336** 484-487). Hybrid p24-Ty VLPs were prepared as disclosed in WO-A-08803562. Macaques were immunised with a single, sterile intramuscular dose of 200μg in the absence of adjuvant. CTL activity was assessed 14 days after immunisation by expanding peripheral blood lymphocytes (PBL) *in vitro* with p24-VLPs or with overlapping gag peptides to create effector cells. PBLs were also stimulated with PHA or ConA and labelled with gag-VDPs or overlapping gag peptides to provide autologous target cells. Incubation of target and effector cells results in the lysis of those target cells where he MHC class I antigen bears peptides corresponding to the CTL epitope.

EXAMPLE 20

Induction of CTL responses by hybrid V3:Ty VLPs

Three potentially important CTL-epitopes of HIV are T1, T2 and Th4.1. The peptide sequences corresponding to these epitopes are:

T1: IKQIINMWQEVGKAMYAP
T2: MHEDIISLWDQSLKPCVK
Th4.1: EGTDRVIEVVQGACRAIR

Hybrid particles of these epitopes were fused in tandem to Ty (T1T2Th4.1:Ty VLPs) were prepared by constructing a vector, pOGS 346. A fusion gene was made by inserting DNA sequences corresponding to these three epitopes in tandem into the *Bam*HI cloning site of pOGS 40. The resulting plasmids were transformed into yeast strains as described in WO-A-8803562. The T1T2Th4.1:Ty VLPs were expressed at high levels, purified by sucrose density gradient and size exclusion chromatography and analysed by SDS/PAGE. Hybrid particles were sterilised and 20µg used to immunise mice by a single intramuscular dose in the absence of adjuvant. Three strains of mice were used for immunisation; Balb/c ($H2^d$), C3H/Hej-($H2^k$) and C57/BL-6 ($H2^b$). Splenocytes are removed after 20 days and expanded by clonal proliferation *in vitro* for 7 to 12 days with synthetic peptides corresponding T1, T2 and Th4.1. The effector cells induced in this way are incubated with target cells of a cognate haplotype which are either control cells or cells labelled with the T1, T2 and Th4.1 peptides, resulting in the lysis of those target cells where the MHC class I antigen bears peptides corresponding to the CTL epitope. Cells whose haplotypes are cognate with the strains of mice used are given in Example 16.

## EXAMPLE 21

### Influenza nucleoprotein constructions

Influenza (strain A PR/8/34) nucleoprotein peptide (residues 147-158) TYQRTRALVTG contains a CTL epitope recognised by BALB/c ($H\text{-}2^d$ haplotype) mice. The R residue has been omitted from the peptide sequence at position 156 as it has been shown that CTL activity is approximately one thousand-fold higher with this residue deleted. Oligonucleotides encoding the nucleoprotein (NP) peptide were cloned as a *Bgl*II-*Bam*HI fragment at the 3' end of yeast *TYA* gene in pOGS 40 yeast expression vector. Sequence verification of these plasmid constructions revealed a single (pOGS 353) and double (pOGS 354) insertion of fragment encoding NP peptide. Both pOGS 353 and 354 were transformed into yeast strain MC2 to generate a Ty:NP fusion. Spin prep analysis of OGS 353 and 354 revealed a good yield of particles. Hybrid Ty-VLPs have been used at various doses to immunise BALB/c mice to assess peptide-specific CTL activity. The NP peptide described above was used to sensitise p815 target cells and to restimulate splenocytes *in vitro*. The induction of influenza-specific CTL was assessed using the previously described assay for detecting V3-specific CTL.

## EXAMPLE 22

### Induction of CTL responses by hybrid Hepatitis B core:HRV antigen particles

Hybrid particles which contain a particle forming sequence from the hepatitis B core antigen and a second sequence containing an epitope from human rhinovirus (HRV) can be made according to the procedure of Brown ***et al***, ***Vaccine*** (1991) **9** 595-601. The neutralisation site from the VP2 protein of HRV-2 is presented within the immunodominant region of the Hep B core particles and the hybrid proteins are used to measure CTL responses in mice. Mice receive a single sterile intramuscular dose in the range of 5-100µg in the absence of adjuvant. Splenocytes are removed after 20 days and expanded by clonal proliferation *in vitro* for 7 to 12 days with peptides corresponding to the HRV epitope. The effector cells induced in this way are incubated with target cells of the cognate haplotype, either control cells or cells labelled with the peptides corresponding to the HRV epitope, resulting in the lysis of those target cells where the MHC class I antigen bears peptides corresponding to the CTL epitope.

## EXAMPLE 23

### Induction of CTL responses by hybrid Hepatitis B core:FMDV antigen particles

Hybrid particles which contain a particle forming sequence from the hepatitis B core antigen and a second sequence containing an epitope from Foot and Mouth Disease Virus (FMDV) can be made according to the procedure of Beesley ***et al***, ***Bio/Technology*** (1990) **8** 64-648. The major immunogenic epitope of FMDV is fused N-terminally to the Hep B core particles and the hybrid proteins are used to measure CTL responses in mice. Mice receive a single sterile intramuscular dose in the range of 5-100µg in the absence of adjuvant. Splenocytes are removed after 20 days and expanded by clonal proliferation *in vitro* for 7 to 12 days with peptides corresponding to the FMDV epitope. The effector cells induced in this way are incubated with target cells of the cognate haplotype, either control cells or cells labelled with the peptides corresponding to the FMDV epitope, resulting in the lysis of those target cells where the MHC class I antigen bears peptides corresponding to the CTL epitope.

## Claims

**1.** A sterile, adjuvant-free pharmaceutical formulation comprising a proteinaceous particulate antigen.

**2.** A formulation as claimed in claim 1, wherein the particulate antigen comprises a plurality of self-assembling protein molecules.

**3.** A formulation as claimed in claim 2, wherein the particulate antigen comprises a homologous population of protein molecules.

**4.** A formulation as claimed in claim 2 or 3, wherein the self-assembling protein molecules each comprise a first amino acid sequence which has the property of self-assembly fused to a second amino acid sequence which has an epitope capable of inducing a cytotoxic T-lymphocyte (CTL) response in a suitable host ("a CTL epitope") and which has the desired antigenic specificity or one of the desired antigenic specificities.

**5.** A formulation as claimed in claim 4, wherein the second amino acid sequence also comprises a sequence whose function is to stimulate the production of CD4 T-cells ("a CD4 T-cell epitope")

**6.** A formulation as claimed in claim 4 or 5, wherein the self-assembling sequence is derived from the yeast retroelement Ty.

**7.** A formulation as claimed in claim 4, 5 or 6, wherein the self-assembling sequence is derived from a virus, preferably from: the gag protein of a retrovirus; hepatitis B virus, surface or core antigen; or bluetongue virus, inner or outer capsid.

**8.** A formulation as claimed in claim 7, wherein the retrovirus is HIV-2, HTLV-I, HTLV-II, HTLV-III, SIV, BIV, ELAV, CIAV, murine leukaemia virus, Moloney murine leukaemia virus or feline leukaemia virus or, preferably, HIV-1.

**9.** A formulation as claimed in any one of claims 4 to 8, wherein the antigenic sequence is derived from a virus protein, the virus optionally being a retrovirus (particularly HIV-1), an orthomyxovirus, a paramyxovirus, a papovavirus, an arenavirus, a hepadnavirus or a herpes virus.

**10.** A formulation as claimed in any one of claims 4 to 8, wherein the antigenic sequence is derived from a protein associated with a tumour, such as a melanoma-associated antigen or an epithelial-tumour associated antigen.

**11.** A formulation as claimed in any one of claims 4 to 8, wherein the antigenic sequence is derived from a parasite, such as *Plasmodium falciparum*.

**12.** A formulation as claimed in any one of claims 4 to 8, wherein the antigenic sequence is derived from a bacterium, such as *Gonococcus*, *Bordetella pertussis*, *Listeria*, *Mycobacteria* or *Leishmania*.

**13.** A formulation as claimed in any one of claims 4 to 12, wherein the second amino acid sequence is derived at least in part from:
1) HIV (particularly HIV-1) gp120,
2) HIV (particularly HIV-1) p24,
3) Influenza virus nucleoprotein,
4) LCMV nucleoprotein,
5) HPV L1 or L2 proteins,
6) p97 melanoma associated antigen,
7) GA 733-2 epithelial tumour-associated antigen,
8) MUC-1 repeat sequence from epithelial tumour-associated antigen,
9) mycobacterium +6 or
10) malaria CSP or RESA antigen.

**14.** The use of a proteinaceous particulate antigen in the preparation of an adjuvant-free pharmaceutical formulation, for inducing a cytotoxic T cell response.

**15.** An immunotherapeutic or prophylactic vaccination kit comprising a first, non-adjuvanted, proteinaceous particulate antigen formulation and a second, adjuvanted, proteinaceous particulate antigen formulation.

**16.** A product comprising a first and second formulations, as defined in claim 15, for combined, simultaneous or sequential administration in the treatment or prophylaxis of disease amenable to immunotherapy or prophylaxis, particularly microbial infection or cancer.

**Claims for the following Contracting States : ES, GR**

**1.** A process for preparing a pharmaceutical formulation, the process comprising sterilising unadjuvanted proteinaceous particulate antigen.

**2.** A process as claimed in claim 1, wherein the particulate antigen comprises a plurality of self-assembling protein molecules.

**3.** A process as claimed in claim 2, wherein the particulate antigen comprises a homologous population of protein molecules.

**4.** A process as claimed in claim 2 or 3, wherein the self-assembling protein molecules each comprise a first amino acid sequence which has the property of self-assembly fused to a second amino acid sequence which has an epitope capable of inducing a cytotoxic T-lymphocyte (CTL) response in a suitable host ("a CTL epitope") and which has the desired antigenic specificity or one of the desired antigenic specificities.

**5.** A process as claimed in claim 4, wherein the second amino acid sequence also comprises a sequence whose function is to stimulate the production of CD4 T-cells ("a CD4 T-cell epitope")

**6.** A process as claimed in claim 4 or 5, wherein the self-assembling sequence is derived from the yeast retroelement Ty.

**7.** A process as claimed in claim 4, 5 or 6, wherein the self-assembling sequence is derived from a virus, preferably from: the gag protein of a retrovirus; hepatitis B virus, surface or core antigen; or bluetongue virus, inner or outer capsid.

**8.** A process as claimed in claim 7, wherein the retrovirus is HIV-2, HTLV-I, HTLV-II, HTLV-III, SIV, BIV, ELAV, CIAV, murine leukaemia virus, Moloney murine leukaemia virus, feline leukaemia virus or, preferably, HIV-1.

**9.** A process as claimed in any one of claims 4 to 8, wherein the antigenic sequence is derived from a virus protein, the virus optionally being a retrovirus (particularly HIV-1), an orthomyxovirus, a paramyxovirus, a papovavirus, an arenavirus, a hepadnavirus or a herpes virus.

**10.** A process as claimed in any one of claims 4 to 8, wherein the antigenic sequence is derived from a protein associated with a tumour, such as a melanoma-associated antigen or an epithelial-tumour associated antigen.

**11.** A process as claimed in any one of claims 4 to 8, wherein the antigenic sequence is derived from a parasite, such as *Plasmodium falciparum*.

**12.** A process as claimed in any one of claims 4 to 8, wherein the antigenic sequence is derived from a bacterium, such as *Gonococcus*, *Bordetella pertussis*, *Listeria*, *Mycobacteria* or *Leishmania*.

**13.** A process as claimed in any one of claims 4 to 12, wherein the second amino acid sequence is derived at least in part from:
1) HIV (particularly HIV-1) gp120,
2) HIV (particularly HIV-1) p24,
3) Influenza virus nucleoprotein,
4) LCMV nucleoprotein,
5) HPV L1 or L2 proteins,
6) p97 melanoma associated antigen,
7) GA 733-2 epithelial tumour-associated antigen,
8) MUG-1 repeat sequence from epithelial tumour-associated antigen,
9) mycobacterium +6 or
10) malaria CSP or RESA antigen.

**14.** The use of a proteinaceous particulate antigen in the preparation of an adjuvant-free pharmaceutical formulation, for inducing a cytotoxic T cell response.

**15.** An immunotherapeutic or prophylactic vaccination kit comprising a first, non-adjuvanted, proteinaceous particulate antigen formulation and a second, adjuvanted, proteinaceous particulate antigen formulation.

**16.** A product comprising a first and second formulations, as defined in claim 15, for combined, simultaneous or sequential administration in the treatment or prophylaxis of disease amenable to immunotherapy or prophylaxis, particularly microbial infection or cancer.

FIG.1A

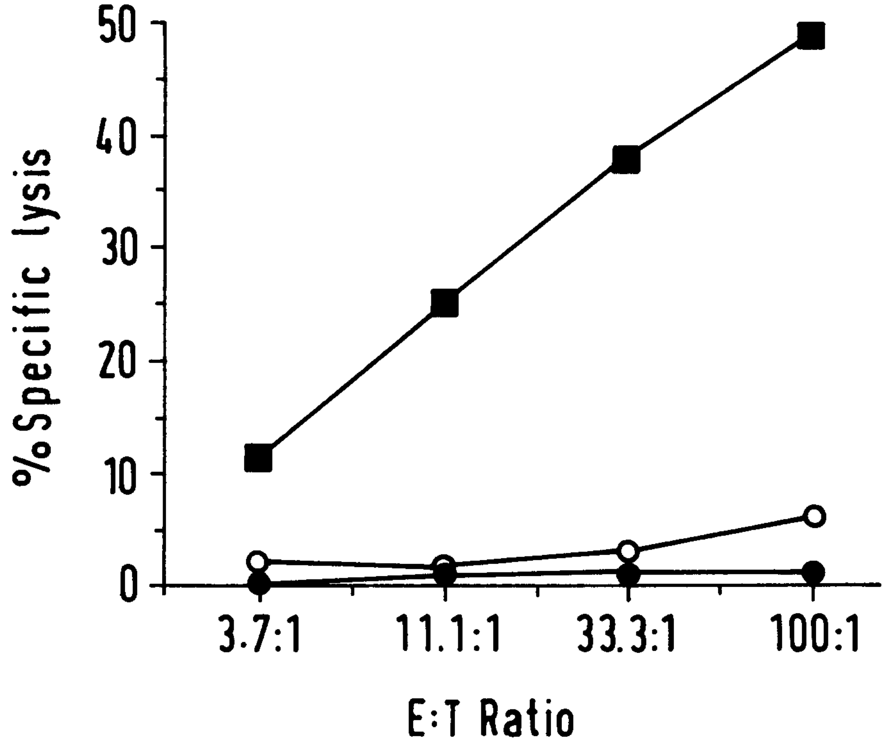
50
40
30
20
10
0
% Specific lysis
3.7:1
11.1:1
33.3:1
100:1
E:T Ratio

FIG.1B

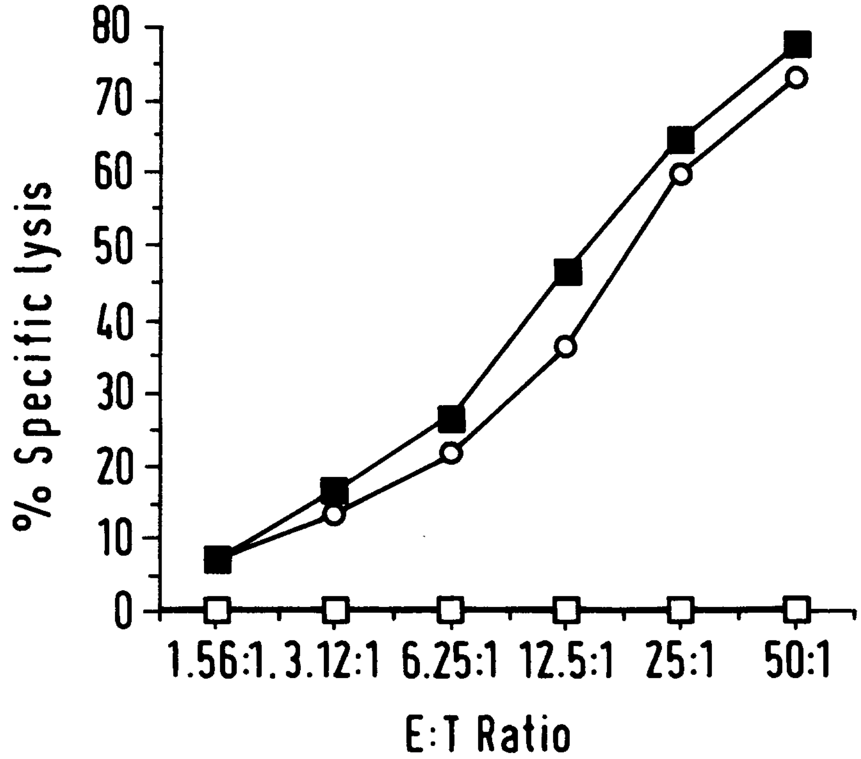
80
70
60
50
40
30
20
10
0
% Specific lysis
1.56:1.
3.12:1
6.25:1
12.5:1
25:1
50:1
E:T Ratio

FIG. 1C

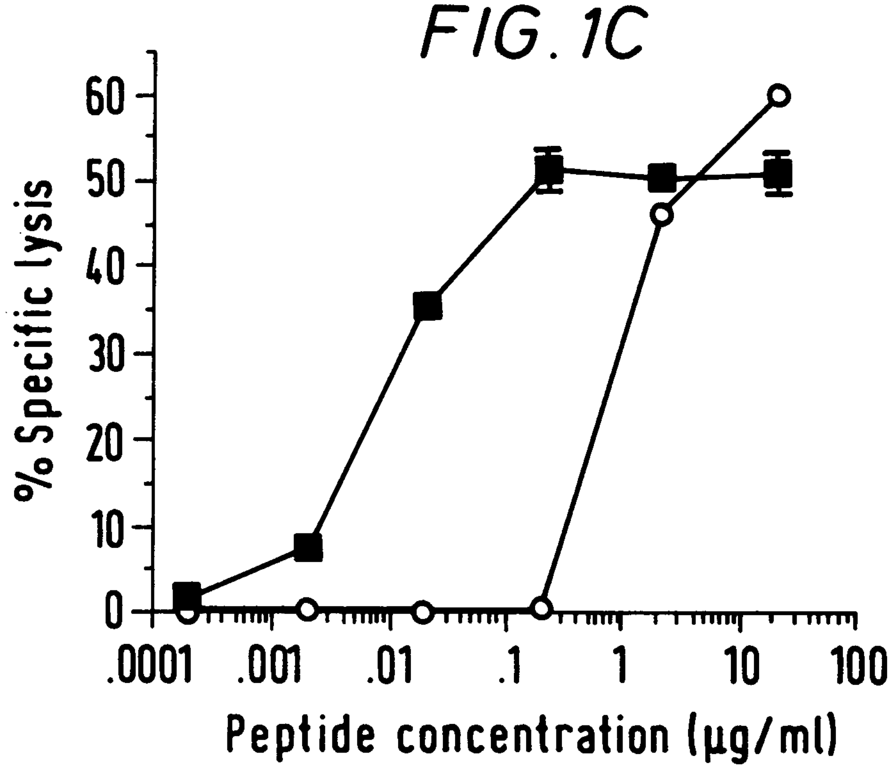
% Specific lysis
60
50
40
30
20
10
0
.0001
.001
.01
.1
1
10
100
Peptide concentration (µg/ml)

FIG. 3

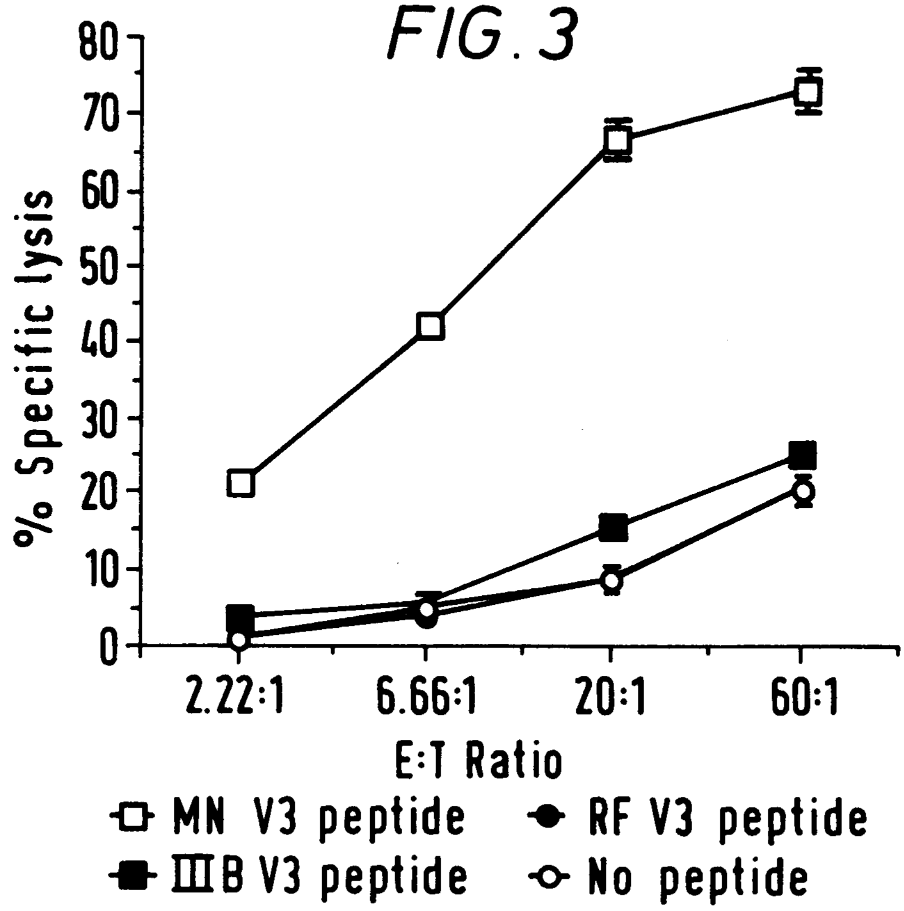
% Specific lysis
80
70
60
50
40
30
20
10
0
2.22:1
6.66:1
20:1
60:1
E:T Ratio
MN V3 peptide
RF V3 peptide
IIIB V3 peptide
No peptide

FIG.2A

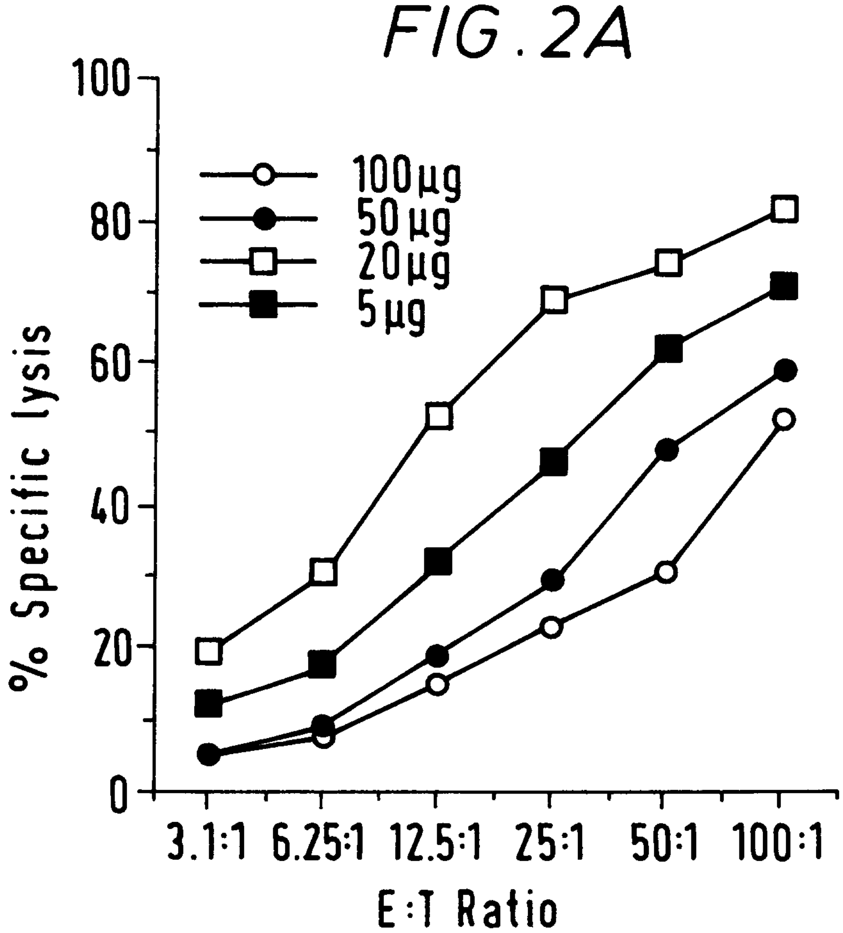
100
80
60
40
20
0
% Specific lysis
100µg
50µg
20µg
5µg
3.1:1
6.25:1
12.5:1
25:1
50:1
100:1
E:T Ratio

FIG.2B

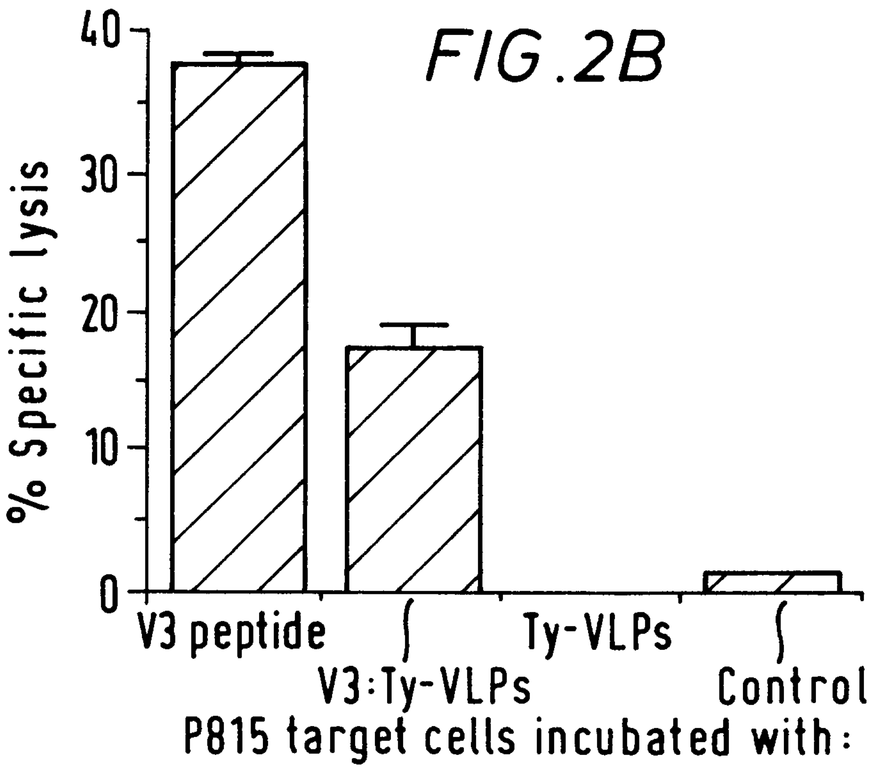
40
30
20
10
0
% Specific lysis
V3 peptide
V3:Ty-VLPs
Ty-VLPs
Control
P815 target cells incubated with:

FIG. 4

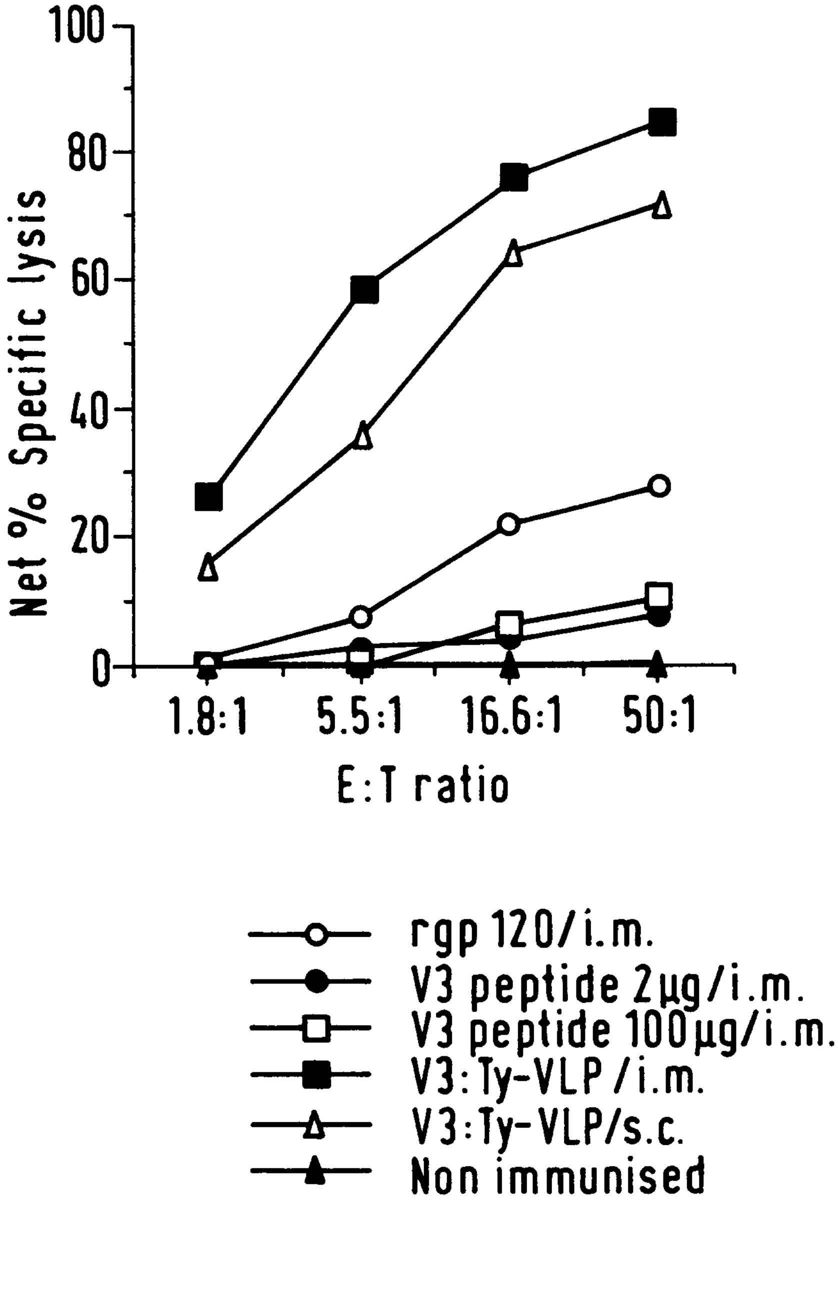
100
80
60
40
20
0
Net % Specific lysis
1.8:1
5.5:1
16.6:1
50:1
E:T ratio
rgp 120/i.m.
V3 peptide 2µg/i.m.
V3 peptide 100µg/i.m.
V3:Ty-VLP/i.m.
V3:Ty-VLP/s.c.
Non immunised

FIG. 5

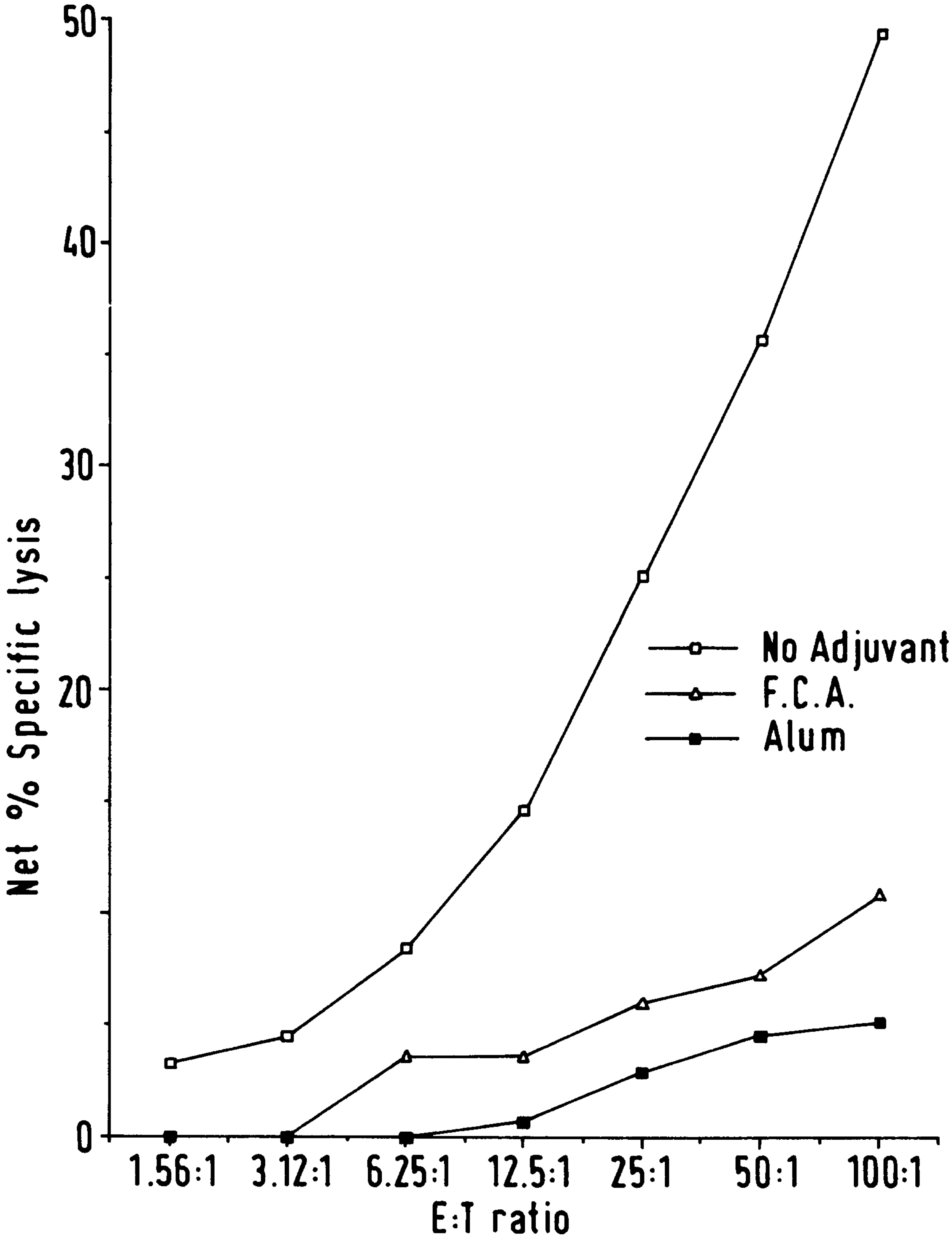
50
40
30
20
0
Net % Specific lysis
No Adjuvant
F.C.A.
Alum
1.56:1
3.12:1
6.25:1
12.5:1
25:1
50:1
100:1
E:T ratio

European Patent Office

# EUROPEAN SEARCH REPORT

Application Number

EP 92 30 7692
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 354 109 (INSTITUT PASTEUR)<br>* page 13, line 36 - line 42; figure 9 * | 1--5,7, 9,13-16 | A61K39/00 |
| X | EP-A-0 425 082 (THE WELLCOME FOUNDATION LTD)<br>* page 10, line 46 - page 11, line 34; table 2 * | 1-5,7, 12,14-16 | |
| X,D | WO-A-8 803 562 (OXFORD GENE SYSTEMS LTD.)<br>* the whole document * | 1-6,9,13 | |
| X | WO-A-9 107 425 (ONCOGEN LTD)<br>* page 66 - page 72 * | 1-5,7-9, 13 | |
| X | EP-A-0 278 940 (SMITH-KLINE RIT)<br>* page 15, line 41 - line 43 *<br>* page 35; example 14 * | 1-5,7,9, 11,13 | |
| X | EP-A-0 449 116 (WOLF)<br>* the whole document * | 1-5,7-9, 13 | TECHNICAL FIELDS SEARCHED (Int. Cl.5 )<br>A61K<br>C12N<br>C07K |
| X | WO-A-9 114 703 (IMPERIAL COLLEGE OF SCIENCE AND TECHNOLOGY)<br>* page 24; example 3 * | 1-3 | |
| X | PROC. NATL. ACAD. SCI. USA<br>vol. 87, 1990,<br>pages 2545 - 2549<br>M. FRANCIS AT AL. 'Immunological properties of hepatitis B core antigen fusion proteins'<br>* the whole document *<br>-/-- | 1-5,7,9, 14-16 | |

The present search report has been drawn up for all claims

| Place of search | Date of completion of the search | Examiner |
|---|---|---|
| THE HAGUE | 14 DECEMBER 1992 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application Number

EP 92 30 7692
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | BIOTECHNOLOGY<br>vol. 6, no. 9, 1988,<br>pages 1065 - 1070<br>T. RUTGERS ET AL. 'Hepatitis B surface antigen as carrier for the repetitive epitope of the circumsporozoite protein of Plasmodium falciparum'<br>* the whole document *<br>--- | 1-5,7,<br>11,13-16 | |
| X | SCIENCE<br>vol. 233, 1986,<br>pages 472 - 475<br>F. DELPEYROUX ET AL. 'A polio virus neutralization epitope expressed on hybrid hepatitis B surface antigen particles'<br>* figure 1 *<br>--- | 1-5,7,<br>13-16 | |
| X | J. VIROL.<br>vol. 64, no. 5, 1990,<br>pages 2452 - 2455<br>M.-L. MICHEL ET AL. 'T and b lymphocyte responses to human immunodeficiency virus type 1 in macaques immunized with hybrid HIV/hepatitis B surface antigen particles'<br>* the whole document *<br>----- | 1-5,7,9,<br>13-16 | TECHNICAL FIELDS SEARCHED (Int. Cl.5 ) |

The present search report has been drawn up for all claims

| Place of search | Date of completion of the search | Examiner |
|---|---|---|
| THE HAGUE | 14 DECEMBER 1992 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document